# EUROPÄISCHE PATENTSCHRIFT

(11) **EP 2 411 811 B1**
(45) Veröffentlichungstag und Bekanntmachung des Hinweises auf die Patenterteilung: **03.08.2016**
(21) Anmeldenummer: 10713156.7
(22) Anmeldetag: 25.03.2010
(51) Int. Cl.: G01N 33/543, B03C 1/00, G01N 35/00

(54) **ÜBERSCHICHTUNG ZUR PARTIKELABTRENNUNG**
LAYERING FOR SEPARATING PARTICLES
SUPERPOSITION DE PHASES POUR LA SÉPARATION DE PARTICULES

(30) Priorität: 25.03.2009 DE 102009001864
(43) Veröffentlichungstag der Anmeldung: 01.02.2012
(60) Teilanmeldung: 16161887.1
(73) Patentinhaber: Qiagen GmbH, 40724 Hilden (DE)
(72) Erfinder: CRAMER, Janina, 40723 Hilden (DE)
(74) Vertreter: Banse & Steglich
(86) Internationale Anmeldenummer: PCT/EP2010/053865
(87) Internationale Veröffentlichungsnummer: WO 2010/108971

(56) Entgegenhaltungen:
- WO-A2-2006/071770
- anonymous: "MagMAXTM Express 96 User Manual" Juni 2008 (2008-06), XP002585996 Gefunden im Internet: URL:http://www.ambion.com/techlib/prot/fm_ 4387988.pdf> [gefunden am 2010-06-07]

## Beschreibung

Gegenstand der Erfindung ist ein Verfahren zur Reinigung von Biomolekülen oder zur Analyse, ob eine wässrige Phase Biomoleküle enthält, wobei aus einer wässrigen Phase, die magnetische Partikel und gegebenenfalls Biomoleküle enthält, die Partikel mit einem magnetischen Stab abgetrennt werden, dadurch gekennzeichnet, dass die wässrige Phase mit einer organischen Phase überschichtet ist, und dass der magnetische Stab bei dem Abtrennen der Partikel aus der wässrigen Phase durch die organische Phase geführt wird. Die Erfindung betrifft auch Verwendungen, Vorrichtungen und Kits, die das erfindungsgemäße Verfahren betreffen.

Nach dem Stand der Technik sind biochemische Reinigungsverfahren bekannt, bei denen Biomoleküle an magnetische Partikel binden. Es werden verschiedene Methoden eingesetzt, um anschließend die magnetischen Partikel mit den daran gebundenen Biomolekülen aus der wässrigen Lösung abzutrennen.

Von der Firma Promega wird ein Reinigungssystem zur Isolierung von DNA aus PCR-Ansätzen unter der Markenbezeichnung "Wizard MagneSil PCR-Clean up System" angeboten. Dabei werden magnetische Partikel zu einem PCR-Ansatz gegeben. Die magnetischen Partikel werden mit einem Magneten, der das Probengefäß von außen kontaktiert, an einer Gefäßseite fixiert. Der wässrige Überstand wird abgetrennt, wobei die Partikel in dem Gefäß verbleiben. Anschließend werden Waschschritte durchgeführt und die DNA in demselben Probengefäß von den Partikeln eluiert.

Auf dem gleichen Prinzip beruht ein Reinigungssystem für DNA aus PCR-Ansätzen, das von Macherey-Nagel unter der Markenbezeichnung "NucleoMag 96 PCR" angeboten wird. Auch hier werden die magnetischen Beads an eine Gefäßwand gebunden, während der Überstand mit einer Pipette abgetrennt wird.

Die WO2006/071770 offenbart Vorrichtungen und Verfahren zur Reinigung von Biomolekülen. Dabei werden magnetische Partikel mit daran gebundenen Biomolekülen mit einem Magneten aus Probengefäßen abgetrennt, wobei der Magnet außerhalb des Probengefäßes angeordnet ist und geführt wird. Zusätzlich erfolgt eine Überschichtung der wässrigen Probe mit einer organischen Phase, wobei die Dicke der Überschichtung nicht näher spezifiziert wird.Eine weitere Vorrichtung und ein entsprechendes Verfahren zur Reinigung von Biomolekülen wird in "MagMAXTM Express 96 User Manual", Juni 2008, Applied Biosystems beschrieben. Dabei werden magnetische Partikel mit daran gebundenen Biomolekülen mit einem Magneten aus Probengefäßen abgetrennt, wobei der Magnet innerhalb des Probengefäßes angeordnet ist und geführt wird, aber keine Überschichtung der wässrigen Probe mit einer organischen Phase erfolgt.

Ein weiteres Verfahren zur Isolierung von Nukleinsäuren aus biologischen Proben unter Verwendung magnetischer Teilchen wird bei einem Verfahren eingesetzt, das in einem Kit, beispielsweise einem QIAsymphony virus/bacteria Kit oder einem QIAsymphony DNA oder RNA Kit etc., von der Firma QIAGEN angeboten wird. Dabei wird eine biologische Probe, die Nukleinsäuren enthält, mit magnetischen Partikeln versetzt, die Nukleinsäure binden. Die Partikel werden anschließend mit einem magnetischen Stab abgetrennt. Der magnetische Stab wird dabei in das Probengefäß getaucht und auf und ab bewegt, um die Partikel vollständig zu binden. Danach wird der Stab mit den daran gebundenen Partikeln und der daran gebundenen Nukleinsäure aus dem Probengefäß gezogen und in ein zweites Probengefäß überführt. In dem zweiten Reaktionsgefäß erfolgt die Elution der Nukleinsäure mit geeigneten wässrigen Lösungen. Anschließend wird der magnetische Stab aus dem Eluat entfernt.
Bei dem bekannten Verfahren der Abtrennung der magnetischen Partikel und der daran gebundenen Biomoleküle mit einem magnetischen Stab besteht das Problem, dass der magnetische Stab in unerwünschter Weise die wässrige Lösung verschleppt. So wird mit dem Stab ein unregelmäßiger Anteil der wässrigen Phase abgetrennt, der den Stab und die daran gebundenen Partikel benetzt. Diese Verschleppung eines Anteils der wässrigen Lösung mit den Beads und dem Magnetstab beruht im Wesentlichen auf physikalischen Phänomenen. Bei der Abtrennung des magnetischen Stabs sammelt sich wässrige Lösung an dem Magnetstab an, bis ein unregelmäßiger Abriss eines Tropfens erfolgt. Weitere Ungenauigkeiten können entstehen, wenn je nach Applikation die Partikel vor der Elution einige Minuten an der Luft getrocknet werden.
Problematisch ist vor allem, dass die Menge der überführten wässrigen Phase unregelmäßig ist. Auf diese Weise werden gleichzeitig unregelmäßige Mengen von weiteren, unerwünschten Bestandteilen aus einer wässrigen Phase abgetrennt. Bei der Elution der zu isolierenden Biomoleküle und der anschließenden Analyse kann das Ergebnis dadurch nicht nur quantitativ sondern auch qualitativ verändert werden. Auch Folgereaktionen oder diagnostische Ergebnisse können verändert und verfälscht werden. Gerade bei sensitiven Applikationen, wie beispielsweise dem Nachweis von Viren und Pathogenen, können die beschriebenen Ungenauigkeiten dazu führen, dass solche Verfahren mit einem magnetischen Stab Schwankungen und Einbußen in der Sensitivität nach sich ziehen. Dies betrifft insbesondere Verfahren bei denen zur möglichst hohen Konzentrierung der Biomoleküle geringe Elutionsvolumen gewählt werden.

Insbesondere bei der Verwendung von Proben mit einer hohen Konzentration von Verunreinigungen, beispielsweise bei Zelllysaten, besteht die Gefahr, dass sich unerwünschte Bestandteile, wie Zellwände, Nukleinsäuren oder Proteine, unspezifisch an die magnetischen Beads anlagern/anhaften bzw. daran binden und in unerwünschter Weise transferiert bzw. verschleppt werden.

Das Problem der Verschleppung eines Teils der wässrigen Lösung stellt sich insbesondere bei der Entfernung des magnetischen Stabs mit daran gebundenen magnetischen Partikeln aus Eluaten, in denen ein vorher mit den magnetischen Partikeln isoliertes Biomolekül enthalten ist. Solche Eluate weisen oft ein relativ geringes Flüssigkeitsvolumen auf, so dass die Verschleppung eines Teils der wässrigen Phase die Konzentration des isolierten Biomoleküls im Eluat deutlich verfälscht.

Der Erfindung liegt die Aufgabe zugrunde, ein Verfahren bereitzustellen, das die beschriebenen Probleme überwindet. Die Genauigkeit der bekannten Verfahren zur Abtrennung von Biomolekülen soll verbessert werden. Insbesondere soll bei einer Vielzahl von Proben eine hohe Reproduzierbarkeit erreicht werden. Erfindungsgemäße Verfahren zur Isolierung von Biomolekülen und zur Analyse, ob Biomoleküle in einer Probe enthalten sind, sollen so wenig wie möglich durch Ungenauigkeiten, beispielsweise der Flüssigkeitsvolumen, beeinträchtigt werden. Die Erfindung soll es ermöglichen, die bekannten Verfahren zu vereinfachen und effizienter durchzuführen.

Das der Erfindung zugrunde liegende Problem wird überraschenderweise gelöst durch Verfahren, Verwendungen und Vorrichtungen mit den Merkmalen der Ansprüche 1 bis 12.

Gegenstand der Erfindung ist ein Verfahren zur Reinigung von Biomolekülen oder zur Analyse, ob eine wässrige Phase Biomoleküle enthält, in einem Probengefäß, das ein Leervolumen von 500 µl bis 3000 µl aufweist, wobei aus einer wässrigen Phase, die magnetische Partikel und gegebenenfalls Biomoleküle enthält, die Partikel mit einem magnetischen Stab abgetrennt werden, wobei die wässrige Phase vollständig mit 100 bis 500 µl einer organischen Phase überschichtet ist, und der magnetische Stab bei dem Abtrennen der Partikel aus der wässrigen Phase durch die organische Phase geführt wird, wobei das Verfahren automatisiert durchgeführt wird.

In einer Ausführungsform der Erfindung sind die Biomoleküle bei Abtrennung der magnetischen Partikel mit dem magnetischen Stab nicht an die magnetischen Partikel gebunden und verbleiben in der wässrigen Phase. Bei dieser Ausführungsform wurden die Biomoleküle bevorzugt in einem vorhergehenden Schritt gebunden an die magnetischen Partikel mit dem magnetischen Stab aus einer ersten wässrigen Phase abgetrennt. Die Biomoleküle wurden dann in die zweite wässrige Phase überführt, in der sie von den magnetischen Partikeln getrennt (eluiert) werden. Diese (zweite) wässrige Phase, die mit der Ölphase überschichtet ist oder wird, ist somit ein Eluat.

In einer weiteren Ausführungsform der Erfindung sind die Biomoleküle bei der Abtrennung der magnetischen Partikel an die magnetischen Partikel gebunden und werden mit den magnetischen Partikeln aus der wässrigen Phase abgetrennt. Bei dieser Ausführungs-form ist die wässrige Phase bevorzugt ein Lysat oder ein Reaktionsansatz, aus dem ein freigesetztes Biomolekül oder ein Reaktionsprodukt abgetrennt werden soll.

Das erfindungsgemäße Verfahren kann sowohl zur Isolierung von Biomolekülen als auch zur Analyse, ob eine Probe Biomoleküle aufweist, eingesetzt werden. Häufig ist bei biochemischen Verfahren, insbesondere Diagnostikverfahren, anfangs nicht bekannt, ob eine Probe das gesuchte Biomolekül enthält oder nicht. Erfindungsgemäß werden auch Verfahren, bei denen das (auf-) zu reinigende Biomolekül nicht erhalten wird, als Verfahren "zur Isolierung" dieses Biomoleküls angesehen, da die anfängliche Intention bei der Durchführung des Verfahrens die Isolierung ist. Erfindungsgemäße Verfahren zur Analyse sind insbesondere diagnostische Verfahren, beispielsweise um zu ermitteln, ob eine Probe Viren, Bakterien oder sonstige Pathogene oder deren Bestandteile, oder Marker, die auf Erkrankungen hindeuten, enthält. Der Ausdruck, dass eine Probe "gegebenenfalls Biomoleküle enthält", bedeutet erfindungsgemäß, dass eine Probe "Biomoleküle enthält oder im Verdacht steht, Biomoleküle zu enthalten".

Der magnetische Stab wird beim Abtrennen der Partikel aus der wässrigen Phase durch die organische Phase geführt. Bei diesem Schritt sind die magnetischen Partikel, gegebenenfalls mit daran gebundenen zu isolierenden Biomolekülen, an den magnetischen Stab gebunden.

Die organische Phase verdrängt bei dem Passieren des magnetischen Stabs die wässrige Phase und verhindert so das "Verschleppen" der wässrigen Phase.

Wenn an die magnetischen Partikel Biomoleküle angelagert bzw. gebunden sind und aus der Lösung abgetrennt werden, wird durch die organische Phase verhindert, dass Verunreinigungen aus der wässrigen Phase mit den an die magnetischen Partikel gebundenen Biomolekülen isoliert werden. Dass anstelle der wässrigen Phase ein geringer Anteil der organischen Phase mit dem Magnetstab überführt wird, stört nachfolgende Reaktionen nicht. Zum einen sammelt sich die organische Flüssigkeit nach Überführung der Partikel in eine andere wässrige Phase auf deren Oberfläche an und kann auf einfache Weise abgetrennt werden, zum anderen enthält die organische Flüssigkeit selbst üblicherweise keine störenden Verunreinigungen. Durch das erfindungsgemäße Verfahren wird die unerwünschte Überführung von wässriger Lösung und Verunreinigungen mit dem magnetischen Stab deutlich verringert. Der Anteil der in der Ausgangslösung verbleibenden wässrigen Phase ist bei mehreren Proben sehr gleichmäßig und deutlich größer als bei herkömmlichen Verfahren. Darüber hinaus werden bei direkter Isolierung von Biomolekülen aus Reaktionsansätzen und Lysaten erfindungsgemäß Eluate höherer Reinheit erhalten. Daher können mit dem erfindungsgemäßen Verfahren unter Verwendung einer organischen Phase im Gegensatz zu bekannten Verfahren zusätzlich Waschschritte eingespart werden.

Ein weiterer Vorteil des erfindungsgemäßen Verfahrens ist es, dass unmittelbar nach Abtrennung der zu isolierenden Biomoleküle mit dem Magnetstab durch eine organische Phase keine weiteren Reaktionen zwischen diesen Molekülen und Bestandteilen einer mitgeschleppten wässrigen Phase möglich sind. Wenn, wie bei bekannten Verfahren, mit dem Magnetstab Reste einer wässrigen Phase verschleppt werden, können dagegen bis zur Elution der Biomoleküle noch Wechselwirkungen oder Reaktionen, beispielsweise zwischen zu isolierenden Nukleinsäuren und mitgeschleppten Proteinen, stattfinden, die die Ergebnisse verfälschen.

Wenn magnetische Partikel mit einem magnetischen Stab aus einem Eluat abgetrennt werden, und dabei eine organische Phase passieren, wird gewährleistet, dass die Menge des Eluats nicht oder nur unwesentlich verändert wird. So werden auch keine Biomoleküle in unerwünschter Weise entfernt.

Mit "Biomoleküle" werden erfindungsgemäß Moleküle bezeichnet, die identisch oder auch ein Gemisch sein können, wie beispielsweise Nukleinsäuren, insbesondere eine DNA oder RNA Fraktion, Proteine, Zucker oder Metabolite. In bevorzugten Ausführungsformen der Erfindung sind die Biomoleküle freie oder gebundene Biopolymere, insbesondere Nukleinsäuren, die bevorzugt natürlich oder *in vitro* behandelt sind, oder Proteine oder Zucker. Unter Biopolymeren werden im Sinne der vorliegenden Erfindung natürlich vorkommende Makromoleküle - wie Nukleinsäuren, Proteine oder Polysaccharide - wie auch synthetisch hergestellte - wie z.B. in Fermentationsprozessen erzeugte - Polymere, die gleiche oder ähnliche Bausteine enthalten wie natürliche Makromoleküle, verstanden. Die Biomoleküle können erfindungsgemäß auch Bestandteile von Komplexen, wie Proteinkomplexen, Viren oder Nukleinsäure/Proteinkomplexen, sein, und als solche isoliert werden.

Die Nukleinsäuren sind bevorzugt natürliche, insbesondere lineare, verzweigte oder zirkuläre Nukleinsäuren, wie DNA und/oder RNA. Die DNA kann beispielsweise genomische DNA oder Plasmid-DNA sein. Die DNA kann auch ein Produkt enzymatischer Reaktionen, beispielsweise amplifizierte DNA aus einem PCR-Ansatz oder Fragmente aus einem Enzymverdau sein. Die RNA kann beispielsweise mRNA oder nichtcodierte RNA, wie z.B. miRNA, rRNA, siRNA, snRNA, snoRNA, piRNA, tRNA, hnRNA oder Ribozyme sein.

Allgemein können Biomoleküle nach bekannten Methoden an die magnetischen Partikel angelagert bzw. gebunden werden, wobei beispielsweise ionische Wechselwirkungen, Wasserstoffbrückenbindungen, hydrophile oder hydrophobe Bindungen Affinitätsbindungen oder kovalente Bindungen genutzt werden können. Zur Vereinfachung wird im Weiteren und im Rahmen dieser Anmeldung für jegliche Art der Anlagerung oder Bindung der Begriff "Binden" verwendet.

Als wässrige Phase kann jede wässrige Lösung eingesetzt werden, die in biochemischen oder diagnostischen Verfahren und Assays eingesetzt wird und Biomoleküle enthält. Die wässrige Lösung kann beispielsweise ein Zell-, Bakterien- oder Gewebelysat, eine Körperflüssigkeit, ein *in vitro* Reaktionsansatz oder eine Fraktion einer dieser Lösungen sein. Allgemein können die wässrigen Lösungen auch durch Reinigungs- und Trennungsschritte aus den genannten Quellen erhalten werden. Geeignete wässrige Lösungen sind auch solche, in denen bekannte biochemische Reaktionen, insbesondere enzymatische Reaktionen, durchgeführt wurden. In einer bevorzugten Ausführungsform ist die wässrige Phase ein Eluat, insbesondere ein solches, das bei Zugabe einer Elutionslösung zu magnetischen Partikeln erhalten wird.

Als organische Phase eignen sich grundsätzlich Flüssigkeiten oder Gemische von Flüssigkeiten, die im Wesentlichen nicht mit Wasser mischbar sind und bei Zugabe zu Wasser eine obere Phase mit einer deutlichen Phasengrenze ausbilden. In bevorzugten Ausführungsformen der Erfindung enthält die organische Phase Kohlenwasserstoffe, insbesondere gesättigte Kohlenwasserstoffe, oder besteht daraus. Geeignete Kohlenwasserstoffe sind verzweigte oder unverzweigte, substituierte oder nicht substituierte, acyclische oder cyclische Kohlenwasserstoffe mit 5, 6, 7, 8, 9, 10, 11, 12, 13, 14, 15, 16, 17, 18, 19 oder 20 Kohlenstoffatomen. Bevorzugt werden verzweigte oder unverzweigte substituierte acyclische oder cyclische Kohlenwasserstoffe mit 6, 7, 8, 9, 10, 11, 12, 13, 14, 15 oder 16 Kohlenstoffatomen. Besonders bevorzugt werden unsubstituierte, acyclische verzweigte oder unverzweigte Kohlenwasserstoffe mit 8 9, 10, 11 oder 12 Kohlenstoffatomen, worunter n-Octan, n-Nonan, n-Decan und Mineralöle ganz besonders bevorzugt werden. Unter Mineralölen werden im Sinne der vorliegenden Erfindung die aus mineralischen Rohstoffen - wie Erdöl, Braun- und Steinkohlen, Holz oder Torf - gewonnen flüssigen Destillationsprodukte verstanden, die im wesentlichen aus Gemischen von gesättigten Kohlenwasserstoffen bestehen (vgl. Römpp, Lexikon Chemie, Thieme Verlag, Stuttgart).

Die organische Phase kann auch Silikonöl enthalten oder daraus bestehen. Siliconöle sind geeignet, da sie in hohem Maße inert gegenüber anderen Substraten sind und ein hohes Spreitungsvermögen zeigen, dass mit der die Ausbildung besonderer Eigenschaften, beispielsweise der Hydrophobie, einhergeht. Geeignete Siliconölen sind insbesondere Syntheseöle auf Basis halborganischer Polymere und Copolymere aus Silicium-Sauerstoff-Einheiten mit organischen Seitenketten. Bevorzugt sind Polymere oder Copolymere aus unverzweigten, wechselweise aus Silicium- und Sauerstoffatomen aufgebauten Ketten. Die Polymere weisen insbesondere Kettenlängen von 10 bis 1000, bevorzugt von 30 bis 500, besonders bevorzugt von 50 bis 150 Siliciumatomen auf.

Die Überschichtung von wässrigen Phasen mit organischen Phasen wurde im Stand der Technik im Bereich der Biochemie bereits beschrieben. Dabei erfolgte die Überschichtung jedoch zu unterschiedlichen Zwecken und in unterschiedlichen Verfahren als bei der vorliegenden Erfindung. Insbesondere war ein Einsatz einer organischen Phase bei Reinigungsverfahren mit magnetischen Beads nicht bekannt. So offenbart die europäische Patentanmeldung EP 1 559 478 A1 den Einsatz von Kohlenwasserstoffen als Kontaminationsbarriere. Durch das Überschichten wässriger Lösungen mit Kohlenwasserstoffen wird bei Verarbeitung von hohen Probenanzahlen, wie beim Hochdurchsatz-Screening, vermieden, dass Verunreinigungen durch Verschleppungen oder Aerosolbildungen entstehen.

Die europäische Patentanmeldung EP 1 256 627 A1 betrifft die Verwendung von Kohlenwasserstoffen zur Vermeidung von Kreuzkontaminationen. Dabei wird bei dem Einsatz von Probengefäßen, die an der Bodenseite eine Auslassöffnung aufweisen, durch die Überschichtung einer Probe mit Alkanen eine gleichmäßigere und vollständigere Abtrennung der wässrigen Phase durch die Auslassöffnung erreicht.

In einer bevorzugten Ausführungsform der Erfindung sind die magnetischen Partikel Silica-Partikel. Diese werden nach dem Stand der Technik insbesondere zur Bindung von Nukleinsäuren eingesetzt. Magnetische Silica-Partikel zur Bindung von Nukleinsäuren sind kommerziell erhältlich und werden beispielsweise von der Firma QIAGEN in Kits der Markenbezeichnung "MagAttract" angeboten. Solche Partikel werden auch als "Teilchen" oder "Beads" bezeichnet. Erfindungsgemäß können jedoch alle bekannten magnetischen Partikel eingesetzt werden, die in der Lage sind, Biomoleküle zu binden.

Als magnetischer Stab ist erfindungsgemäß jede längliche Vorrichtung einsetzbar, die geeignet ist, bei Einführung in ein Probengefäß magnetische Partikel aus dem Probengefäß abzutrennen. Die Bindung der magnetischen Partikel an den magnetischen Stab kann an einem Magnetstabschutz erfolgen, der auf den Magnetstab aufgesetzt ist.

In einer Ausführungsform der Erfindung wird der Stab durch die wässrige und die organische Phase geführt. In einer weiteren Ausführungsform wird der Stab nur durch die organische Phase geführt, ohne mit der flüssigen Phase in Kontakt zu treten. In einer weiteren Ausführungsform wird der Stab durch die organische Phase und den Grenzbereich der Phasen geführt, wobei er die flüssige Phase in der Eintauchrichtung zu nicht mehr als 2 %, 3 %, 4 %, 5%, 6 %, 10 %, 15 % oder 20% durchläuft. Es wurde beobachtet, dass die Verschleppung der wässrigen Phase verringert werden kann, wenn die flüssige Phase nicht vollständig durchlaufen wird.

Erfindungsgemäß erfolgt nach der Entfernung der magnetischen Partikel mit daran gebundenen Biomolekülen aus der Lösung bevorzugt die weitere Aufreinigung, Analyse oder Verarbeitung nach bekannten Methoden. Diese umfassen Elution der Biomoleküle, Waschund Reinigungsschritte, Analyse der Art und Menge, und Durchführung weitere Reaktionen. Nach der Abtrennung (Elution) der Biomoleküle von den magnetischen Partikeln folgen erfindungsgemäß bevorzugt die weitere Aufreinigung, Analyse oder Verarbeitung nach bekannten Methoden. Diese umfassen Wasch- und Reinigungsschritte, Analyse der Art und Menge, und Durchführung weitere Reaktionen.

Die wässrige Phase liegt in einem Probengefäß vor, das ein Leervolumen von 500 µl bis 3000 µl aufweist. Bevorzugt sind Leervolumen zwischen 500 µl und 1500 µl. Dies entspricht üblichen Probengefäßen, die im Labormaßstab zur Untersuchung oder Bearbeitung einer Vielzahl von Proben eingesetzt werden. Das Volumen der wässrigen Phase in der Probe liegt beispielsweise zwischen 10 µl und 1000 µl, insbesondere zwischen 50 µl und 750 µl.

Die organische Phase wird in einer Menge von 100 µl bis 500 µl eingesetzt. Die Menge an organischer Phase wird so ausgewählt, dass die wässrige Phase vollständig überschichtet ist. Bei der Verwendung üblicher Probengefäße im Labormaßstab ("Eppendorf-Cups") sind insbesondere mehr als 200µl oder mehr als 250µl geeignet. Bevorzugt ist insbesondere die Verwendung einer Menge zwischen 200 µl und 400 µl oder zwischen 250 µl und 350 µl. Die Dicke der organischen Phase ist bevorzugt an der dünnsten Stelle mindestens 1 mm, 2 mm, 3 mm, 4 mm oder 5 mm.

In einer Ausführungsform der Erfindung wird nach der Zugabe der magnetischen Partikel zu der wässrigen Phase und/oder der Überschichtung der wässrigen Phase mit der organischen Phase und vor der Abtrennung der Partikel mit dem magnetischen Stab keine chemische, insbesondere keine enzymatische Reaktion, wie beispielsweise eine PCR-Reaktion, durchgeführt. Dies bedeutet, dass chemische Reaktionen in diesem Zeitraum nicht initiiert werden, beispielsweise durch Zugabe von Reagenzien wie Enzymen. Es kann natürlich nicht ausgeschlossen werden, dass vor diesem Zeitraum eingeleitete Reaktionen noch in geringem Maße weiter erfolgen. Allerdings kann eine Reaktion aktiv durchgeführt werden, welche die Ablösung der Biomoleküle von den magnetischen Partikeln auslöst oder unterstützt.

In einer Ausführungsform der Erfindung wurde in der wässrigen Phase vor Durchführung des Verfahrens keine PCR-Reaktion durchgeführt und/oder das zu isolierende Biomolekül ist kein PCR-Produkt. Bei der bekannten PCR-Reaktion erfolgt die Überschichtung von Proben mit Mineralöl, um das Verdampfen der wässrigen Lösungen bei den eingesetzten hohen Temperaturen zu verhindern. Nach der Durchführung einer PCR-Reaktion nach dem Stand der Technik wird das Amplifikat jedoch nicht mit einem Magnetstab durch das Mineralöl transferiert, sondern die wässrige Phase wird zunächst unter der organischen Phase abpipettiert und das Mineralöl verworfen.

Erfindungsgemäß können zu der wässrigen Phase zunächst die magnetischen Partikel gegeben werden und anschließend die Probe mit der organischen Phase überschichtet werden. Erfindungsgemäß ist es jedoch auch möglich, die wässrige Phase zunächst mit der organischen Phase zu überschichten und erst anschließend die magnetischen Partikel hinzuzufügen, beispielsweise durch pipettieren direkt in die wässrige Phase.

In einer weiteren Ausführungsform der Erfindung findet nach der Überschichtung der wässrigen Phase mit der organischen Phase und/oder der Hinzufügung der magnetischen Partikel eine Reaktion, beispielsweise eine enzymatische Reaktion wie eine PCR-Reaktion, statt. Beispielsweise kann eine PCR-Reaktion unter üblichen Bedingungen durchgeführt werden, wobei der PCR-Ansatz mit einer organischen Phase überschichtet ist. Nach Abschluss der Reaktion werden dem PCR-Ansatz die magnetischen Partikel hinzugefügt, um die DNA zu binden. Die Partikel mit der daran gebundenen DNA werden anschließend mit dem magnetischen Stab durch die organische Phase abgetrennt. Bei dieser Ausführungs-form der Erfindung dient die organische Phase sowohl als Verdampfungsbarriere bei der PCR-Reaktion als auch zur Verbesserung der Genauigkeit der Abtrennung der DNA aus der Lösung.

Das Verfahren wird automatisiert durchgeführt.

Das erfindungsgemäße Verfahren zur Reinigung von Biomolekülen aus einer wässrigen Phase oder zur Analyse, ob eine wässrige Phase Biomoleküle enthält, umfasst insbesondere folgende Schritte:
(a) Bereitstellen einer ersten wässrigen Phase, die Biomoleküle enthält, in einem ersten Reaktionsgefäß,
(b) Zugabe magnetischer Partikel,
(c) Abtrennen der magnetischen Partikel mit daran gebundenen Biomolekülen aus der wässrigen Phase mit einem magnetischen Stab,
(d) Überführen der magnetischen Partikel mit den daran gebundenen Biomolekülen in ein zweites Reaktionsgefäß,
(e) Elution der Biomoleküle von den magnetischen Partikeln in dem zweiten Reaktionsgefäß in einer zweiten wässrigen Phase,
(f) Abtrennen der magnetischen Partikel mit dem magnetischen Stab aus dem zweiten Reaktionsgefäß, wobei die Biomoleküle in der (zweiten) wässrigen Phase verbleiben,
wobei vor Schritt (c) die erste wässrige Phase vollständig mit 100 bis 500 µl einer organischen Phase überschichtet wird und der magnetische Stab bei dem Abtrennen der Partikel aus der ersten wässrigen Phase durch die organische Phase geführt wird, wobei das erste Reaktionsgefäß ein Leervolumen von 500 µl bis 3000 µl aufweist, und/oder vor Schritt (f) die zweite wässrige Phase vollständig mit 100 bis 500 µl einer organischen Phase überschichtet wird und dass der magnetische Stab bei dem Abtrennen der Partikel aus der zweiten wässrigen Phase durch die organische Phase geführt wird, wobei das zweite Reaktionsgefäß ein Leervolumen von 500 µl bis 3000 µl aufweist,
wobei das Verfahren automatisiert durchgeführt wird.

Die Verfahrensschritte werden in der Reihenfolge (a) bis (f) durchlaufen. Die Zugabe magnetischer Partikel und das Überschichten der wässrigen Phase erfolgt nach bekannten Methoden, beispielsweise mit einer Pipette. Danach kann die wässrige Phase inkubiert werden. Nach Schritt (b) wird ein magnetischer Stab in die erste wässrige Phase eingeführt. Der Stab wird bevorzugt bewegt, um eine vollständige Bindung der magnetischen Partikel zu erreichen.

Vor Schritt (c) werden in dem ersten Reaktionsgefäß Bedingungen eingestellt, unter denen die Biomoleküle an die magnetischen Partikel binden (Schritt (b1)). Die Bindung erfolgt bevorzugt selektiv. In Schritt (c) und (d) wird der Stab mit den Partikeln und den daran gebundenen Biomolekülen abgetrennt und in ein zweites Reaktionsgefäß überführt, in dem die Elution (e) der Biomoleküle erfolgt. Zur Elution (e) werden Bedingungen eingestellt, die die Wechselwirkung zwischen den magnetischen Partikeln und den daran gebundenen Biomolekülen beeinträchtigen. Im Falle von an Silica-Partikel gebundenen Nukleinsäuren erfolgt die Elution beispielsweise durch Veränderung der Salzkonzentration. Vor der Elution der Biomoleküle (e) können einer oder mehrere Waschschritte durchgeführt werden.

Nach dem Stand der Technik sind Verfahren mit den Schritten (a) bis (f) ohne Überschichten einer wässrigen Lösung mit organischer Phase bekannt. Ein solches Verfahren wird beispielsweise bei dem Kit verwendet, der von der Firma QIAGEN unter der Bezeichnung "QIAsymphony DNA Kit " angeboten wird. Auf das in dem zugehörigen Handbuch (Mai 2008) beschriebene Verfahren wird hier ausdrücklich Bezug genommen. In mindestens einem nachgeschalteten Schritt (g) kann beispielsweise die Analyse folgen, ob die Biomoleküle im Eluat enthalten sind. Es können auch weitere Reinigungsschritte oder Folgereaktionen folgen.

Gegenstand der Erfindung ist auch die Verwendung einer organischen Flüssigkeit, die nicht mit Wasser mischbar ist, zur Verbesserung der Genauigkeit eines automatisierten Verfahrens, bei dem Biomoleküle, die an magnetische Partikel gebunden sind, mit einem magnetischen Stab aus einer wässrigen Phase abgetrennt werden, wobei die wässrige Phase vollständig mit 100 bis 500 µl der organischen Flüssigkeit überschichtet ist, und der magnetische Stab bei dem Abtrennen der Partikel aus der wässrigen Phase durch die organische Phase geführt wird, in einem Probengefäß, das ein Leervolumen von 500 µl bis 3000 µl aufweist. Das erfindungsgemäße Verfahren bewirkt insbesondere, dass gleichmäßige und nur sehr geringe Mengen von wässriger Lösung mit dem magnetischen Stab aus der Ausgangslösung abgetrennt werden. Die Verbesserung der Genauigkeit liegt somit in einem besonders gleichmäßigen Volumen der überschichteten wässrigen Lösung, insbesondere eines Eluats, und in der Verringerung des Anteils an Verunreinigungen.

Gegenstand der Erfindung ist auch eine Vorrichtung zur automatisierten Reinigung von Biomolekülen aus einer wässrigen Phase oder zur Analyse, ob eine wässrige Phase Biomoleküle enthält, umfassend mindestens ein Reaktionsgefäß, das ein Leervolumen von 500 µl bis 3000 µl aufweist, enthaltend eine wässrige Lösung, Biomoleküle und magnetische Partikel, wobei die wässrige Lösung vollständig mit 100 bis 500 µl einer organischen Phase überschichtet ist, die mit Wasser nicht mischbar ist, wobei die Vorrichtung einen magnetischen Stab aufweist, der so ausgebildet ist, dass er bei dem Abtrennen der Partikel aus der wässrigen Phase durch die organische Phase geführt wird. Die Vorrichtung ist beispielsweise ein Laborroboter oder eine automatisierte Einrichtung, die eine Vielzahl von Proben untersuchen kann.

Die Erfindung ist verwendbar in Zusammenhang mit einem Kit zur Reinigung von Biomolekülen mit einem magnetischen Stab aus einer wässrigen Phase oder zur Analyse, ob eine wässrige Phase Biomoleküle enthält, umfassend eine wässrige Lösung, Biomoleküle, magnetische Partikel und eine organischen Flüssigkeit, die nicht mit Wasser mischbar ist. Das Kit enthält bevorzugt einen magnetischen Stab.

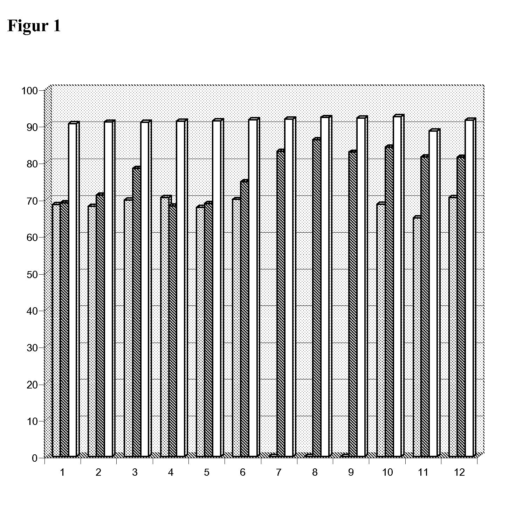

Figur 1 zeigt die Ergebnisse der Ausführungsbeispiele 1 bis 12. Für jedes Beispiel zeigt der linke Balken (hellgrau) das Elutionsvolumen in µl ohne Ölüberschichtung, der mittlere Balken (dunkelgrau) das Elutionsvolumen in µl mit Ölüberschichtung und der rechte Balken (weiß) das eingesetzte Ausgangsvolumen in µl (Inputvolumen). Bei den Messergebnissen handelt es sich jeweils um Durchschnittswerte über 8 Proben eines Prozessgefäßes (Prozesstubes).
Beispiele 1-3: 10 µl, 50 µl bzw. 200 µl Öl mit Separation über die Ölschicht.
Beispiele 4-6: 10 µl, 50 µl bzw. 200 µl Öl mit Separation im Bereich der wässrigen Phase.
Beispiele 7-9: dreifache Bestimmung mit jeweils 300 µl Ölüberschichtung und Separation nur über die Ölschicht.
Beispiele 10 - 12: dreifache Bestimmung mit jeweils 300 µl Ölüberschichtung und Separation über die gesamte Flüssigkeit.

### Ausführungsbeispiele

### Durchführung

Die Ermittlung des Einflusses einer Ölüberschichtung bei der Trennung von magnetischen Partikeln von einer nukleinsäurehaltigen Lösung erfolgte halbautomatisch auf einem automatisierten Probenpräparationsmodul (QIAsymphony™ SP, Firma Qiagen). Hierzu wurde ein verkürzter Ablauf auf dem System wie folgt programmiert:

Zunächst wurden die auf dem Gerät eingesetzten 8er Prozessgefäße (Sample Prep Cartridges, Firma QIAGEN) mit Hilfe der Pipettiereinheit mit jeweils 300 µl Puffer befüllt. Anschließend wurden die magnetischen Beads im entsprechenden Reservoir zum Erhalt einer homogenen Suspension gemischt und jeweils 40 µl der Suspension zu dem bereits vorgelegten AVE Puffer (einem handelsüblichen Wasch- und/oder Elutionspuffer, Firma QIAGEN) hinzugefügt. Die so entstandene Suspension wurde mit Hilfe der Mischeinheit auf dem Gerät für 10 sec. gemischt und anschließend die magnetischen Partikel mit den in der Mischeinheit vorhandenen Magnetstäbe und dem aufgesetzten und zuvor gewogenem Magnetstabschutz (8-Rod Cover, Firma QIAGEN) durch mehrmaliges Durchfahren der Flüssigkeitssäule am Magnetstabschutz gesammelt und aus der Flüssigkeit gehoben. Die am Magnetstabschutz gesammelten magnetischen Partikel wurden durch 8-minütiges Verweilen an der Luft getrocknet. Parallel wurde ein weiteres, zuvor leer gewogenes Prozessgefäß mit dem eigentlichen Elutionspuffer AVE von 91 µl vorbereitet und zur Ermittlung des durchschnittlichen Vorlagevolumens gewogen. Nach der Trocknung wurden die magnetischen Partikel von dem Magnetstabschutz in den vorgelegten Elutionspuffer transferiert. Es erfolgte ein weiterer 3-minütiger Mischschritt mit Hilfe der Mischeinheit des Gerätes. Nach der so durchgeführten Elution wurden die magnetischen Partikel erneut an den Magnetstabschutz gesammelt (Separation) und das im Prozessgefäß verbliebene Eluat durch Wägung ermittelt. Anschließend wurden die zuvor am Magnetstabschutz gesammelten magnetischen Partikel wieder vollständig in das Eluat überführt. Danach wurde die Suspension manuell jeweils mit der gleichen Menge Mineralöl überschichtet, gewogen und erneut separiert. Nach der Separation wurde das Prozessgefäß zur Ermittlung der nun zurückgehaltenen Elutionsmenge gewogen. Der Magnetstabschutz mit den daran befindlichen magnetischen Partikeln wurde in ein frisches, zuvor gewogenes Prozessgefäß abgeworfen und ebenfalls gewogen. Um die im Prozessgefäß zurückgehaltene Eluatmenge mit Ölüberschichtung genau ermitteln zu können, wurden die Prozessgefäße in einem Trockenschrank bei etwa 35°C gelagert. Nach vollständigem Abdampfen der wässrigen Phase erfolgte die Ermittlung des Gewichts bis zur Konstanz.

Zunächst sollte der Einfluss der Ölmenge auf das wiedergewonnene Eluat geprüft werden. Hierzu wurden 10 µl, 50 µl und 100 µl Mineralöl auf die jeweiligen Eluate eines Prozessgefäßes pipettiert. Die drei Prozessgefäße mit jeweils 8 Eluaten wurden simultan durch den oben beschriebenen Prozess geführt. Hierbei erfolgte die Separation der Beads, indem die Flüssigkeitssäule der wässrigen Phase (91 µl) vom Gefäßboden ausgehend vollständig durchfahren wurde. In einem weiteren Versuch wurde mit der gleichen Versuchsanordnung etwa die Hälfte der Flüssigkeitssäule der wässrigen Phase (50 µl) vom Gefäßboden ausgehend während der Separation durchfahren.

In einem dritten Versuch sollte der Einfluss der Art der Separation weiter untersucht werden. Hierzu erfolgte eine Veränderung des in der Software verankerten Ablaufs der Separation. Dabei sollte die Separation nicht wie üblich über die Flüssigkeitssäule vom Boden aus gesehen erfolgen, sondern nur über die Ölphase. Bei diesem Versuch wurde für die vollständige Überschichtung des eingesetzten Elutionsvolumen ein Ölvolumen von 300 µl eingesetzt und die Separation über die Flüssigkeitssäule des Öls mit einem Offset vom Boden von 96 µl (wässrige Phase) bis zu einer maximalen Füllhöhe von 421 µl durchgeführt. Des Weiteren verweilte der Magnetstabschutz mit den eingefahrenen Magnetstäben, für 3 sec. am oberen Punkt der Separation (421 µl) um möglichst viele der magnetischen Partikel aus der wässrigen Phase durch die gesamte Flüssigkeitssäule des Öls zu ziehen.

Aufgrund der Problematik eines Eingriffes in die regulären Abläufe der Separation wurde in einem weiteren Versuch mit einem Ölvolumen von 300 µl die komplette Flüssigkeitssäule vom Gefäßboden bis zu einem oberen Punkt der Flüssigkeitssäule von 421 µl durchfahren.

### Ergebnisse

### Beispiele 1 bis 6

Die Beispiele 1, 2, 4 und 5 sind Vergleichsbeispiele.

In einer ersten Versuchsreihe wurde der Einfluss der Ölmenge auf die Verdrängung der wässrigen Phase aus den magnetischen Partikeln analysiert (Fig. 1, Beispiele 1-3). Die Separation erfolgte dabei über die gesamte Flüssigkeitssäule bestehend aus wässriger Phase und Ölphase. Es konnte deutlich erkannt werden, dass das Öl in geringen Mengen von 10 µl oder 50 µl nur einen marginalen Einfluss auf die zurückgehaltene Eluatmenge (wässrige Phase) hat (mittlere dunkelgraue Säulen). Die zurückgehaltene Eluatmenge ohne Ölschicht (linke hellgraue Säulen) entsprach den Elutionsmengen, die bei der nach dem Stand der Technik bekannten Elution ohne Ölschicht zu erwarten war. Größere Ölmengen bewirkten hingegen eine signifikante Erhöhung des zurückgehaltenen Volumens (s. insbesondere Fig. 1, Beispiele 3 / mittlere dunkelgraue Säule).

In einer zweiten Versuchsreihe wurden ebenfalls unterschiedliche Ölvolumen gewählt. Zudem wurde die Separation nur im Bereich der wässrigen Phase durchgeführt (Fig. 1, Beispiele 4-6). Hier zeigte sich tendenziell das gleiche Ergebnis, jedoch waren die Effekte weniger stark ausgeprägt.

Die beiden Versuchsreihen zeigten deutlich, dass dem Ölvolumen eine besondere Rolle zukommt. Beobachtungen der Abläufe während der Separation deuteten an dass eine geschlossene Öldecke über der wässrigen Phase einen sehr positiven Einfluss auf die Verdrängung der wässrigen Phase aus den magnetischen Partikeln hat. Bei geringer Ölmenge wird die wässrige Phase durch die Oberflächenspannung mit den magnetischen Partikeln beim Herausfahren des Magnetstabschutzes mit nach oben gerissen und das Öl verliert seinen positiven Effekt auf die Verdrängung der wässrigen Phase aus den magnetischen Partikeln.

### Beispiele 7 bis 9

In einer dritten Versuchsreihe wurde das Ölvolumen deutlich erhöht, um eine geschlossene Ölphase über der wässrigen Phase zu erhalten und um den Effekt auf die anschließende Separation der magnetischen Partikel durch die Ölschicht hindurch nutzen zu können. Um diesen Effekt besonders zu verstärken, sollte die Separation im Wesentlichen über die Ölschicht erfolgen. Hierzu wurde der Ablauf auf dem Gerät für die Separation so verändert, dass der Magnetstabschutz mit eingefahrenen Magnetstäben sich nur durch die Ölphase bewegt und nur geringen direkten Kontakt zur wässrigen Phase bekam. Darüber hinaus wurde auch der Magnetstabschutz mit eingefahrenen Magnetstäben vor der eigentlichen Separationsbewegung für 3 s an der Öloberfläche belassen, um ein erstes Ansammeln der magnetischen Partikel an der Grenze zwischen Öl- und wässriger Phase zu bewirken. Da die Messung nur über die Ölphase erfolgte, war bei diesem Versuch keine direkte Referenzmessung ohne Ölphase möglich. Das Ergebnis in (Fig. 1, Beispiele 7-9) zeigt einen signifikanten Anstieg der zurückgehaltenen Eluatmenge (etwa 91% des eingesetzten Elutionsvolumens). Nur ein geringer Anteil geht durch die Anhaftung an die magnetischen Partikel verloren. Im Vergleich zur herkömmlichen Elution ohne Ölüberschichtung konnte eine Steigerung von etwa 15 % beim wiedergefundenen Eluat verzeichnet werden.

### Beispiele 10 bis 12

Aufgrund der Schwierigkeit, dass in den Beispielen 7 bis 9 spezielle Separationsbedingungen benötigt wurden, wurde in den weiteren Beispielen 10 bis 12 bei einer herkömmlichen Separation eine erhöhte Ölmenge (300 µl) eingesetzt. Wie bei den Beispielen 7 bis 9 wurde vor der eigentlichen Separationsbewegung eine Verweildauer des Magnetstabschutzes an der Öloberfläche von 3 s vorgesehen. Die Ergebnisse zeigen eine geringfügige Verringerung der zurückgehaltenen Eluatmenge (Fig. 1: Beispiele 10-12). Es konnten etwa 90 % des Eluats zurückgehalten werden.

Die Versuche zeigen, dass die Überschichtung einer mit magnetischen Partikeln angereicherten wässrigen Phase mit Mineralöl dabei hilft, bei der Abtrennung der magnetischen Partikel mittels eines Magnetstabes die wässrige Phase weitestgehend aus den magnetischen Partikeln zu verdrängen. Hierbei spielen insbesondere zwei Effekte eine Rolle. Zum Einen verhindert die Ölschicht, dass Teile der wässrigen Phase durch die asymmetrische Ansammlung des magnetische Partikel (Beadpellet) nicht über Kapillarkräfte in der wässrigen Phase zwischen dem herausragendem Beadpellet und der Gefäßwand nach oben verschleppt werden und im ungünstigsten Fall einen Tropfenabriss bewirken, so dass die wässrige Lösung am Beadpellet verbleibt und somit verloren geht. Zum Anderen bewirkt die Ölschicht eine Verdrängung der wässrigen Phase aus den magnetischen Partikeln. Dieser positive Effekt ist besonders ausgeprägt, wenn die Ölmenge für eine geschlossene Überschichtung der wässrigen Phase ausreicht. Noch etwas bessere Resultate werden erzielt, wenn während der Separation der magnetischen Partikel durch die Ölschicht der Magnetstab nicht die wässrige Phase zum besseren Einsammeln der restlichen Partikel durchfährt.

## Patentansprüche

1. Verfahren zur Reinigung von Biomolekülen oder zur Analyse, ob eine wässrige Phase Biomoleküle enthält,
in einem Probengefäß, das ein Leervolumen von 500 µl bis 3000 µl aufweist,
wobei aus einer wässrigen Phase, die magnetische Partikel und gegebenenfalls Biomoleküle enthält, die Partikel mit einem magnetischen Stab abgetrennt werden, **dadurch gekennzeichnet, dass** die wässrige Phase vollständig mit 100 bis 500 µl einer organischen Phase überschichtet ist, und dass der magnetische Stab bei dem Abtrennen der Partikel aus der wässrigen Phase durch die organische Phase geführt wird, wobei das Verfahren automatisiert durchgeführt wird.

2. Verfahren nach Anspruch 1, wobei die Biomoleküle beim Abtrennen der magnetischen Partikel mit dem magnetischen Stab nicht an die magnetischen Partikel gebunden sind und in der wässrigen Phase verbleiben.

3. Verfahren nach Anspruch 1 oder 2, wobei in einem vorhergehenden Schritt aus einer ersten wässrigen Phase die Biomoleküle gebunden an die magnetischen Partikel mit dem magnetischen Stab abgetrennt wurden.

4. Verfahren nach Anspruch 1, wobei die Biomoleküle bei der Abtrennung der magnetischen Partikel an die magnetischen Partikel gebunden sind und mit den magnetischen Partikeln aus der wässrigen Phase abgetrennt werden.

5. Verfahren nach einem der vorhergehenden Ansprüche, wobei die Biomoleküle Nukleinsäuren, Proteine oder Zucker sind oder enthalten.

6. Verfahren nach einem der vorhergehenden Ansprüche, wobei die wässrige Phase ein Zell-, Bakterien- oder Gewebelysat, eine Körperflüssigkeit, ein *in vitro* Reaktionsansatz oder eine Fraktion einer dieser Lösungen, oder ein Eluat ist.

7. Verfahren nach einem der vorhergehenden Ansprüche, wobei die organische Phase Kohlenwasserstoffe und/oder Silikonöl enthält.

8. Verfahren nach einem der vorhergehenden Ansprüche, wobei die magnetischen Partikel Silica-Partikel sind.

9. Verfahren nach einem der vorhergehenden Ansprüche, wobei nach der Zugabe der magnetischen Partikel zu der wässrigen Phase und/oder nach der Überschichtung der wässrigen Phase mit der organischen Phase und vor der Abtrennung der Partikel mit dem magnetischen Stab keine chemische, insbesondere keine enzymatische, Reaktion durchgeführt wird.

10. Verfahren zur Reinigung von Biomolekülen aus einer wässrigen Phase oder zur Analyse, ob eine wässrige Phase Biomoleküle enthält, umfassend die Schritte:
(a) Bereitstellen einer ersten wässrigen Phase, die gegebenenfalls Biomoleküle enthält, in einem ersten Reaktionsgefäß,
(b) Zugabe magnetischer Partikel,
(c) Abtrennen der magnetischen Partikel mit daran gebundenen Biomolekülen aus der ersten wässrigen Phase mit einem magnetischen Stab,
(d) Überführen der magnetischen Partikel mit den daran gebundenen Biomolekülen in ein zweites Reaktionsgefäß,
(e) Elution der Biomoleküle von den magnetischen Partikeln in dem zweiten Reaktionsgefäß in eine zweite wässrige Phase,
(f) Abtrennen der magnetischen Partikel mit dem magnetischen Stab aus dem zweiten Reaktionsgefäß, wobei die Biomoleküle in der wässrigen Phase verbleiben,
**dadurch gekennzeichnet, dass** vor Schritt (c) die erste wässrige Phase vollständig mit 100 bis 500 µl einer organischen Phase überschichtet wird und der magnetische Stab bei dem Abtrennen der Partikel aus der ersten wässrigen Phase durch die organische Phase geführt wird, wobei das erste Reaktionsgefäß ein Leervolumen von 500 µl bis 3000 µl aufweist,
und/oder
dass vor Schritt (f) die zweite wässrige Phase vollständig mit 100 bis 500 µl einer organischen Phase überschichtet wird und dass der magnetische Stab bei dem Abtrennen der Partikel aus der zweiten wässrigen Phase durch die organische Phase geführt wird, wobei das zweite Reaktionsgefäß ein Leervolumen von 500 µl bis 3000 µl aufweist,
wobei das Verfahren automatisiert durchgeführt wird.

11. Verwendung einer organischen Flüssigkeit, die nicht mit Wasser mischbar ist, zur Verbesserung der Genauigkeit eines automatisierten Verfahrens, bei dem Biomoleküle, die an magnetische Partikel gebunden sind, mit einem magnetischen Stab aus einer wässrigen Phase abgetrennt werden, wobei die wässrige Phase vollständig mit 100 bis 500 µl der organischen Flüssigkeit überschichtet ist, und der magnetische Stab bei dem Abtrennen der Partikel aus der wässrigen Phase durch die organische Phase geführt wird, in einem Probengefäß, das ein Leervolumen von 500 µl bis 3000 µl aufweist.

12. Vorrichtung zur automatisierten Reinigung von Biomolekülen aus einer wässrigen Phase oder zur Analyse, ob eine wässrige Phase Biomoleküle enthält, umfassend mindestens ein Reaktionsgefäß, das ein Leervolumen von 500 µl bis 3000 µl aufweist, enthaltend eine wässrige Lösung, Biomoleküle und magnetische Partikel, wobei die wässrige Lösung vollständig mit 100 bis 500 µl einer organischen Phase überschichtet ist, die mit Wasser nicht mischbar ist, wobei die Vorrichtung einen magnetischen Stab aufweist, der so ausgebildet ist, dass er bei dem Abtrennen der Partikel aus der wässrigen Phase durch die organische Phase geführt wird.

## Claims

1. A process for the purification of biomolecules or for the analysis, whether an aqueous phase comprises biomolecules,
in a sample vessel, which has a void volume of from 500 µl to 3000 µl,
wherein from an aqueous phase, which comprises magnetic particles and possibly biomolecules, the particles are separated with a magnetic rod,
**characterized in that** the aqueous phase is completely overlaid with 100 to 500 µl of an organic phase, and that the magnetic rod is guided through the organic phase during the separation of the particles from the aqueous phase, wherein the process is carried out automatically.

2. A process according to claim 1, wherein the biomolecules are not bound to the magnetic particles during the separation of the magnetic particles using the magnetic rod, and remain in the aqueous phase.

3. A process according to claim 1 or 2, wherein in a preceding step the biomolecules bound to the magnetic particles were separated from a first aqueous phase with the magnetic rod.

4. A process according to claim 1, wherein the biomolecules are bound to the magnetic particles during the separation of the magnetic particles and are separated from the aqueous phase with the magnetic particles.

5. A process according to any of the preceding claims, wherein the biomolecules are or comprise nucleic acids, proteins, or sugars.

6. A process according to any of the preceding claims, wherein the aqueous phase is a cell, bacteria or tissue lysate, a body fluid, an *in vitro* reaction mixture, or a fraction of one of said solutions, or an eluate.

7. A process according to any of the preceding claims, wherein the organic phase comprises hydrocarbons and/or silicone oil.

8. A process according to any of the preceding claims, wherein the magnetic particles are silica particles.

9. A process according to any of the preceding claims, wherein after the addition of the magnetic particles to the aqueous phase and/or after the overlaying of the aqueous phase with the organic phase and before the removal of the particles using the magnetic rod, no chemical reaction, more particularly no enzymatic reaction is carried out.

10. A process for purifying biomolecules from an aqueous phase or for analyzing, whether an aqueous phase comprises biomolecules, comprising the steps of:
(a) providing a first aqueous phase, which possibly comprises biomolecules, in a first reaction vessel,
(b) adding magnetic particles,
(c) separating the magnetic particles with biomolecules bound thereto from the first aqueous phase using a magnetic rod,
(d) transferring the magnetic particles with the biomolecules bound thereto into a second reaction vessel,
(e) eluting the biomolecules from the magnetic particles in the second reaction vessel into a second aqueous phase,
(f) separating the magnetic particles from the second reaction vessel using the magnetic rod, wherein the biomolecules remain in the aqueous phase, **characterized in that** prior to step (c) the first aqueous phase is overlaid completely with 100 to 500 µl of an organic phase and the magnetic rod is guided through the organic phase during the separation of the particles from the first aqueous phase, wherein the first reaction vessel has a void volume from 500 µl to 3000 µl,
and/or
that prior to step (f) the second aqueous phase is overlaid completely with 100 to 500 µl of an organic phase and the magnetic rod is guided through the organic phase during the separation of the particles from the second aqueous phase, wherein the second reaction vessel has a void volume from 500 µl to 3000 µl,
wherein the process is carried out automatically

11. The use of an organic liquid, which is immiscible with water, for the improvement of the accuracy of an automated process, by which biomolecules, which are bound to magnetic particles, are separated with a magnetic rod from the aqueous phase, wherein the aqueous phase is completely overlayed with 100 to 500µl of the organic liquid, and the magnetic rod is guided through the organic phase during the separation of the particles from the aqueous phase, in a sample vessel, which has a void volume from 500 µl to 3000 µl.

12. An apparatus for the automated purification of biomolecules from an aqueous phase or for analyzing whether an aqueous phase comprises biomolecules, comprising at least one reaction vessel, which has a void volume of from 500 µl to 3000 µl, containing an aqueous solution, biomolecules, and magnetic particles, wherein the aqueous solution is completely overlaid with 100 to 500 µl of an organic phase, which is immiscible with water, wherein the apparatus has a magnetic rod, which is configured for being guided through the organic phase during the separation of the particles from the aqueous phase.

## Revendications

1. Procédé de purification de biomolécules ou d'analyse pour déterminer si une phase aqueuse contient des biomolécules,
dans un tube à essais, qui présente un volume vide de 500 µl à 3 000 µl,
selon lequel, à partir d'une phase aqueuse qui contient des particules magnétiques et éventuellement des biomolécules, les particules sont séparées avec un barreau magnétique,
**caractérisé en ce que** la phase aqueuse est recouverte en totalité avec 100 à 500 µl d'une phase organique, et **en ce que** le barreau magnétique traverse la phase organique lors de la séparation des particules de la phase aqueuse, le procédé étant réalisé sous forme automatisée.

2. Procédé selon la revendication 1, dans lequel, lors de la séparation des particules magnétiques avec le barreau magnétique, les biomolécules ne sont pas reliées aux particules magnétiques et restent dans la phase aqueuse.

3. Procédé selon la revendication 1 ou 2, dans lequel, lors d'une étape précédente, les biomolécules ont été séparée d'une première phase aqueuse reliées aux particules magnétiques avec le barreau magnétique.

4. Procédé selon la revendication 1, dans lequel, lors de la séparation des particules magnétiques, les biomolécules sont reliées aux particules magnétiques et sont séparées de la phase aqueuse avec les particules magnétiques.

5. Procédé selon l'une quelconque des revendications précédentes, dans lequel les biomolécules sont ou contiennent des acides nucléiques, des protéines ou des sucres.

6. Procédé selon l'une quelconque des revendications précédentes, dans lequel la phase aqueuse est un lysat cellulaire, bactérien ou tissulaire, un fluide corporel, une préparation réactionnelle *in vitro* ou une fraction d'une de ces solutions, ou un éluat.

7. Procédé selon l'une quelconque des revendications précédentes, dans lequel la phase organique contient des hydrocarbures et/ou une huile de silicone.

8. Procédé selon l'une quelconque des revendications précédentes, dans lequel les particules magnétiques sont des particules de silice.

9. Procédé selon l'une quelconque des revendications précédentes, dans lequel, après l'ajout des particules magnétiques à la phase aqueuse et/ou après le recouvrement de la phase aqueuse avec la phase organique et avant la séparation des particules avec le barreau magnétique, aucune réaction chimique, notamment enzymatique, n'est réalisée.

10. Procédé de purification de biomolécules à partir d'une phase aqueuse ou d'analyse pour déterminer si une phase aqueuse contient des biomolécules, comprenant les étapes suivantes :
(a) la préparation d'une première phase aqueuse, qui contient éventuellement des biomolécules, dans un premier récipient de réaction,
(b) l'ajout de particules magnétiques,
(c) la séparation des particules magnétiques avec les biomolécules reliées à celles-ci à partir de la première phase aqueuse avec un barreau magnétique,
(d) le transfert des particules magnétiques avec les biomolécules reliées à celles-ci dans un second récipient de réaction,
(e) l'élution des biomolécules des particules magnétiques dans le second récipient de réaction dans une seconde phase aqueuse,
(f) la séparation des particules magnétiques avec le barreau magnétique à partir du second récipient de réaction, les biomolécules restant dans la phase aqueuse,
**caractérisé en ce qu'**avant l'étape (c), la première phase aqueuse est recouverte en totalité avec 100 à 500 µl d'une phase organique et le barreau magnétique traverse la phase organique lors de la séparation des particules à partir de la première phase aqueuse, le premier récipient de réaction présentant un volume vide de 500 µl à 3 000 µl,
et/ou
**en ce qu'**avant l'étape (f), la seconde phase aqueuse est recouverte en totalité avec 100 à 500 µl d'une phase organique, et **en ce que** le barreau magnétique traverse la phase organique lors de la séparation des particules à partir de la seconde phase aqueuse, le second récipient de réaction présentant un volume vide de 500 µl à 3 000 µl,
le procédé étant réalisé sous forme automatisée.

11. Utilisation d'un liquide organique, qui n'est pas miscible avec l'eau, pour améliorer la précision d'un procédé automatisé, selon lequel des biomolécules qui sont reliées à des particules magnétiques sont séparées d'une phase aqueuse avec un barreau magnétique, la phase aqueuse étant recouverte en totalité avec 100 à 500 µl du liquide organique, et le barreau magnétique traversant la phase organique lors de la séparation des particules à partir de la phase aqueuse, dans un tube à essais qui présente un volume vide de 500 µl à 3 000 µl.

12. Dispositif pour la purification automatisée de biomolécules à partir d'une phase aqueuse ou pour l'analyse pour déterminer si une phase aqueuse contient des biomolécules, comprenant au moins un récipient de réaction, qui présente un volume vide de 500 µl à 3 000 µl, contenant une solution aqueuse, des biomolécules et des particules magnétiques, la solution aqueuse étant recouverte en totalité avec 100 à 500 µl d'une phase organique qui n'est pas miscible avec l'eau, le dispositif comprenant un barreau magnétique qui est configuré pour traverser la phase organique lors de la séparation des particules de la phase aqueuse.
